# EUROPEAN PATENT APPLICATION

(11) **EP 3 109 726 A1**
(43) Date of publication of application: **28.12.2016**
(21) Application number: 16174980.9
(22) Date of filing: 17.06.2016
(51) Int. Cl.: G06F 1/14, A61N 1/02

(54) **VITAL SIGNS INFORMATION MEASURING APPARATUS**

(30) Priority: 26.06.2015 JP 2015128209
(71) Applicant: Nihon Kohden Corporation, Shinjuku-ku Tokyo 161-8560 (JP)
(72) Inventor: Kashiwabara, Satoshi, Tokyo, 161-8560 (JP)
(74) Representative: Hoffmann Eitle

(57) **Abstract**

A vital signs information measuring apparatus includes: an internal clock; a first correcting section which is configured to execute first time correction on the internal clock by using time information, and which is configured to store a time correction amount in a storage section, the time correction amount indicating a degree of deviation of a time of the internal clock at a timing when the first time correction is performed by the first correcting section; and a second correcting section which, in a case where it is detected that the first correcting section fails to normally correct the time of the internal clock at a prescribed timing, is configured to perform second time correction on the internal clock by using a history of the time correction amount stored in the storage section.

## Description

### BACKGROUND

The presently disclosed subject matter relates to an apparatus for measuring vital signs information (hereinafter, often referred to as a vital signs information measuring apparatus).

Many types of medical equipment which are recently used incorporate a clock (internal clock) which measures the time. Such medical equipment stores measurement values of various vital signs parameters, and times which are measured by the internal clock while associating them with each other. Therefore, the medical equipment displays various measurement values and waveforms together with times.

In the case where the internal clock of the medical equipment fails to correctly measure the time, there is a possibility that the condition of the patient cannot be correctly known. Therefore, adjustment (correction) of the time of the internal clock is a very important process.

Hereinafter, techniques relating to time adjustment (time correction) in medical equipment will be described. JP-T-2013-502632 discloses a system for synchronizing a patient monitoring device with a central server. In the system, the central server transmits a timestamp to the patient monitoring device in response to a data packet transmitted from the patient monitoring device. The patient monitoring device performs correction (synchronization) on an internal clock by using the timestamp.

JP-A-11-253411 discloses a method of adjusting the time of a vital signs information measuring apparatus. In the method, when a blood pressure monitor communicates with an external computer, data of the current date and time (time setting data) are transmitted from the external computer to the blood pressure monitor. Then, the blood pressure monitor corrects the current date and time of an internal clock based on the time setting data.

In the related-art techniques disclosed in JP-T-2013-502632 and JP-A-11-253411, as described above, a medical apparatus corrects the time by using time information which is managed by a server. According to the related-art techniques, however, a situation may arise where, in the case where a network failure or the like occurs, the internal clock operates while the time correction remains not to be done. In this case, there arises a problem in that the medical apparatus cannot be operated by using correct time information. This problem may arise not only in the case where the time information on the server is used, but also in the case where the user corrects the time periodically and manually (including the case where setting is performed through short-range communication, in addition to the case where setting is performed through button operations). Namely, this problem commonly arises in cases where time correction is not performed for any cause.

### SUMMARY

The presently disclosed subject matter may provide a vital signs information measuring apparatus which, even in the case where time correction is not performed for any reason, can correct an internal clock.

The vital signs information measuring apparatus may comprise: an internal clock; a first correcting section which is configured to execute first time correction on the internal clock by using time information, and which is configured to store a time correction amount in a storage section, the time correction amount indicating a degree of deviation of a time of the internal clock at a timing when the first time correction is performed by the first correcting section; and a second correcting section which, in a case where it is detected that the first correcting section fails to normally correct the time of the internal clock at a prescribed timing, is configured to perform second time correction on the internal clock by using a history of the time correction amount stored in the storage section.

### BRIEF DESCRIPTION OF THE DRAWINGS

Fig. 1 is a block diagram illustrating the configuration of a vital signs information measuring apparatus of Embodiment 1.
Fig. 2 is a view illustrating an example of data stored in a storage section 15 in Embodiment 1.
Fig. 3 is a view illustrating the concept of time correction by a second correcting section 19 in Embodiment 1.
Fig. 4 is a view illustrating the concept of the time correction by the second correcting section 19 in Embodiment 1.
Fig. 5 is a view illustrating an example of data stored in the storage section 15 in Embodiment 1.
Fig. 6 is a view illustrating an example of a display screen displayed on a displaying section 16 in Embodiment 1.
Fig. 7 is a view illustrating a display screen of an external apparatus in Embodiment 1.
Fig. 8 is a flowchart illustrating a process flow of a controller 13 in Embodiment 1.

### DETAILED DESCRIPTION OF EXEMPLARY EMBODIMENTS

### <Embodiment 1>

Hereinafter, an embodiment of the presently disclosed subject matter will be described with reference to the drawings. A vital signs information measuring apparatus 1 is an apparatus for measuring vital signs information of the subject, and, for example, a patient monitor, a medical telemeter, an electrocardiograph, or an electromyograph.

The vital signs information measuring apparatus 1 has a vital signs signal inputting section 11, a transmitter/receiver 12, a controller 13, an internal clock 14, a storage section 15, a displaying section 16, and an operating section 17.

The vital signs signal inputting section 11 is electrically connected to electrodes, transducers, or the like attached to the subject, and acquires vital signs signals which are output from them, and which relate to, for example, an electrocardiogram, the respiration, the blood pressure, or the pulse wave. The vital signs signal inputting section 11 supplies the vital signs signals to the controller 13.

The transmitter/receiver 12 transmits various data (measurement values, measured waveforms, and the like relating to various vital signs information) to external apparatuses. Moreover, the transmitter/receiver 12 receives various data from external apparatuses, and supplies the received data to the controller 13. That is, the transmitter/receiver 12 has both a function of a transmitter which transmits data, and that of a receiver which receives data. The communication method which is to be performed by the transmitter/receiver 12 includes network communication such as the Internet, and short-range communication, acoustic communication, electromagnetic communication, and the like in which NFC (Near Field Communication) is implemented. Namely, the transmitter/receiver 12 may be requested to transmit and receive data to and from external apparatuses, and an arbitrary communication method may be employed as the data transmission and reception method.

The internal clock 14 is incorporated in the vital signs information measuring apparatus 1, and measures the time. For example, the internal clock 14 is configured by a crystal oscillator, peripheral circuits, and the like. A first correcting section 18 or a second correcting section 19 which will be described later applies time correction to the internal clock 14.

The storage section 15 is a memory device which stores various data including time correction amounts that will be described later. For example, the storage section 15 may be an incorporated nonvolatile memory (e.g., a hard disk drive), a USB (Universal Serial Bus) memory which is configured so as to be attachable to and detachable from the vital signs information measuring apparatus 1, or the like. The storage section 15 has a concept including also a temporary storage device (for example, a cache memory) which is used by a CPU.

The displaying section 16 is a displaying device which is disposed on the housing of the vital signs information measuring apparatus 1, such as an LCD (Liquid Crystal Display) or a CRT (Cathode Ray Tube). Measurement values (the respiration rate, the blood pressure, the body temperature, etc.), vital waveforms, and the like of various vital signs information are displayed on the displaying section 16. Also the current time which is measured by the internal clock 14 is displayed on the displaying section 16.

The operating section 17 is configured by, for example, buttons disposed on the housing of the vital signs information measuring apparatus 1, or a keyboard which is connectable to the vital signs information measuring apparatus 1. The user inputs operation instructions such as start and stop of measurement of various vital signs information (such as the body temperature, the SpO2, and the blood pressure) by operating the operating section 17. The user may further input correct time information for correcting the time, through the operating section 17.

The displaying section 16 and the operating section 17 may have a form in which they are integrated with each other (namely, a form similar to a touch display).

The controller 13 performs various controls on the vital signs information measuring apparatus 1. The processes of the controller 13 are realized by the CPU (Central Processing Unit) which reads programs stored in the storage section 15, and which executes the programs, and various control circuits.

The controller 13 has the first correcting section 18 and the second correcting section 19. The first correcting section 18 performs time correction on the internal clock 14 at a prescribed timing (for example, 14 o'clock every day) by using time information. The time information is information indicating the correct time which is used in the correction of the time of the internal clock 14. The time information may be manually input through the operating section 17, or input from an external apparatus through the transmitter/receiver 12. For example, a medical person (technologist or the like) may input time information at a predetermined time, or an external apparatus may transmit time information to the vital signs information measuring apparatus 1 at a predetermined time.

The prescribed timing is a timing when the first correcting section 18 periodically corrects the time, and may be a time interval of a certain width (for example, from 14:00 to 15:00 every day). Alternatively, the user may change the prescribed timing. For example, the user may explicitly change the time when time information is acquired from an external apparatus through network.

At the timing when the time correction is performed, the first correcting section 18 calculates a time correction amount indicating the degree of deviation (time deviation) of the time measured by the internal clock 14 which has not yet been corrected, from the correct time (time information). It is assumed that, for example, the time indicated by the internal clock 14 is 14:00:20 of May 25, 2015, and the correct time is 14:00:00 of May 25, 2015. The first correcting section 18 calculates the difference of the two times, i.e., +20 seconds as the time correction amount. Then, the first correcting section 18 stores the calculated time correction amount in the storage section 15.

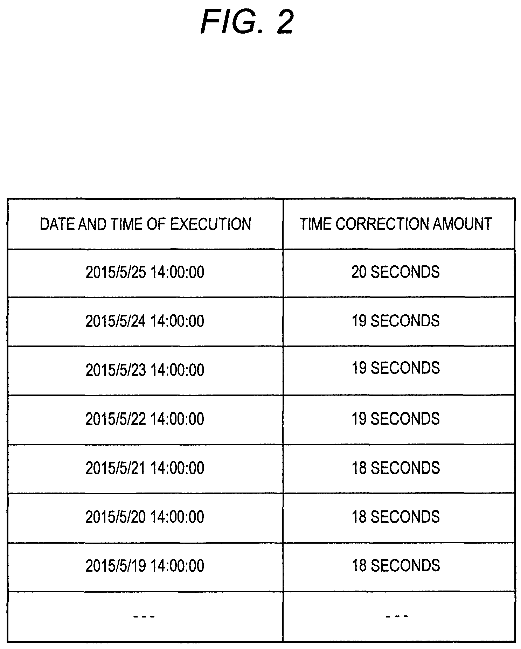

Fig. 2 is a view illustrating an example of the time correction amount stored in the storage section 15. In the example, the first correcting section 18 stores the date and time when the time is corrected (the date and time indicated by the time information), and the time correction amount, in the storage section 15 while associating them with each other. For example, Fig. 2 illustrates that the storage section 15 stores data indicating that the time correction amount in May 25 is 20 seconds, that the time correction amount in May 24 is 19 seconds, and the like. The storage section 15 can store any kind of data as far as the data allow the time correction amount to be calculated. That is, the first correcting section 18 may store, in the storage section 15, the time which has not yet been corrected (e.g., 14:00:20 of May 25, 2015), and the time which has been corrected (e.g., 14:00:00 of May 25, 2015), as they are. The first correcting section 18 may store, in the storage section 15, in place of the date and time when the time is corrected, the elapsed time period (e.g., 24 hours) from the previous time correction, and the time correction amount (e.g., + 20 seconds), while associating them with each other.

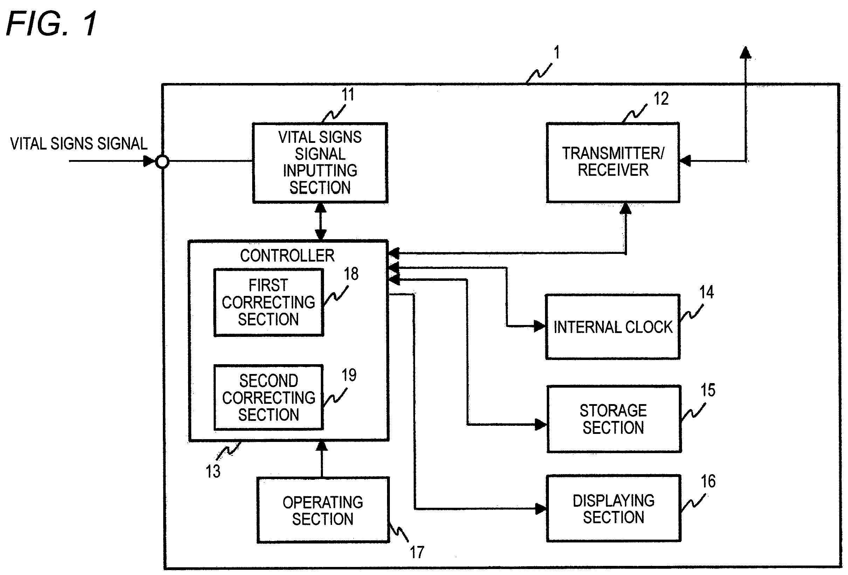

Referring again to Fig. 1, the second correcting section 19 detects whether the first correcting section 18 normally performed the time correction at the prescribed timing or not. For example, the case where the time correction is not normally performed includes the case where the time information is not input through the operating section 17, that where the time information is not normally input through the transmitter/receiver 12 because of a network failure, and that where the time correction process itself fails to operate due to some cause. In the case where the first correcting section 18 fails to normally perform the time correction at the prescribed timing, the second correcting section 19 corrects the time of the internal clock 14 by using the history of the time correction amount stored in the storage section 15. Examples of the time correction by the second correcting section 19 will be described.

A first example will be described in which the time correction amount of the time correction that is most recently performed by the first correcting section 18 is used as it is. In the case where the time correction amounts illustrated in Fig. 2 have been stored in the storage section 15, for example, the second correcting section 19 uses the time correction amount (20 seconds) in May 25, 2015 as the time correction amount in May 26, 2015. Namely, the second correcting section 19 performs correction to shift the time of the internal clock 14 by 20 seconds. The tendency of the deviation of the time of the internal clock 14 is not largely varied. When the most recent time correction amount is used, therefore, the second correcting section 19 can accurately correct the time of the internal clock 14 without substantially performing a calculation process.

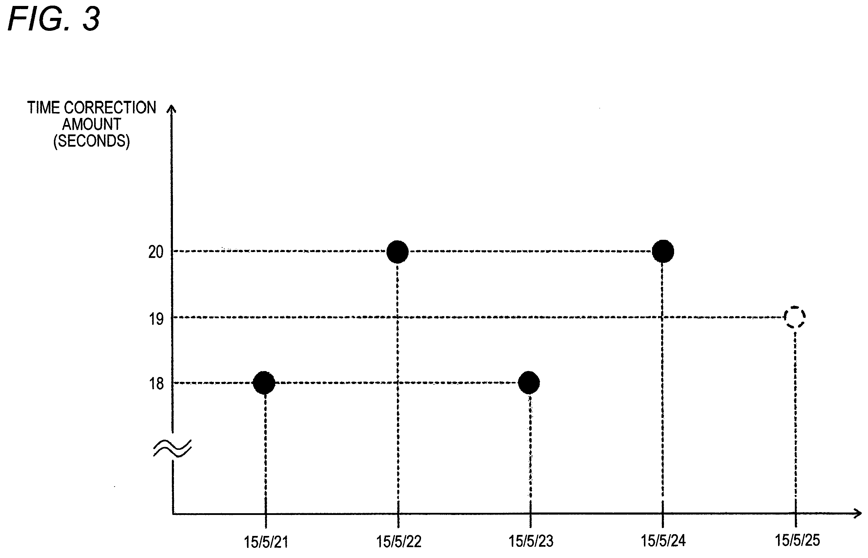

A second example will be described in which the average of a plurality of time correction amounts is used. Fig. 3 is a view illustrating the concept of the operation of the second example. In the example of Fig. 3, the time correction amounts in May 21 and 23, 2015 are 18 seconds, and those in May 22 and 24, 2015 are 20 seconds. The second correcting section 19 obtains the average of time correction amounts in a fixed time period (in the example of Fig. 3, four days). In the example of Fig. 3, the second correcting section 19 calculates the average of the time correction amounts as 19 seconds. Then, the second correcting section 19 performs correction to shift the time of the internal clock 14 by 19 seconds. When the average of the time correction amounts for the internal clock 14 is used as described above, the time of the internal clock 14 can be corrected in accordance with the tendency of the deviation of the time of the internal clock 14.

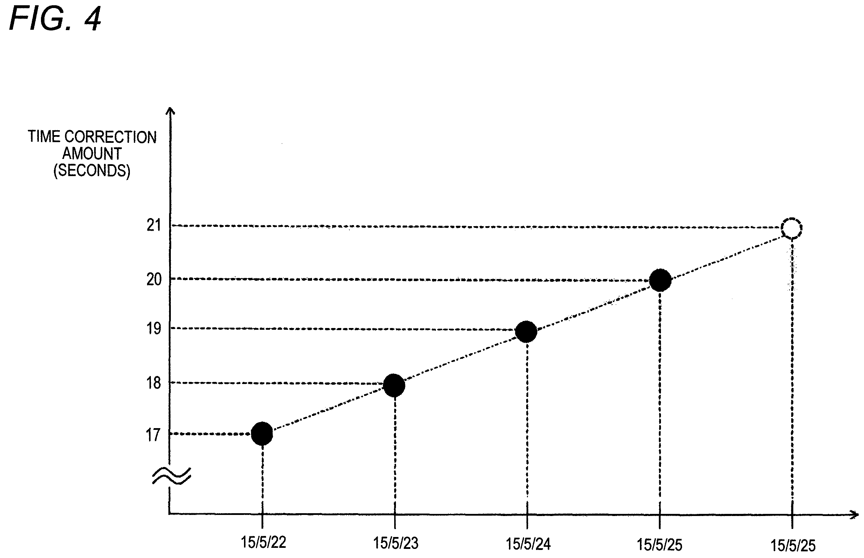

A third example will be described in which a relational expression indicating a relationship between the elapsed time period and the time deviation of the internal clock 14 is calculated. Fig. 4 is a view illustrating the concept of the operation of the third example. The first correcting section 18 stores a time correction amount and the date and time when the time correction is performed, in the storage section 15 while associating them with each other. In the example of Fig. 4, the time correction amount in May 22, 2015 is 17 seconds, that in May 23, 2015 is 18 seconds, that in May 24, 2015 is 19 seconds, and that in May 25, 2015 is 20 seconds. Alternatively, the first correcting section 18 may store the elapsed time period from the previous time correction by the first correcting section 18, and the time correction amount, in the storage section 15 while associating them with each other. That is, the first correcting section 18 may store information relating to the timing when the time is corrected by the first correcting section 18, in the storage section 15 while associating the information with the time correction amount.

From these data, the second correcting section 19 calculates a relational expression indicating a relationship between the elapsed time period and the degree of the deviation of the time of the internal clock 14. The relational expression may be calculated by using a usual method of calculating a regression line. For example, the second correcting section 19 calculates a linear equation of (17 + x) (where x represents the elapsed days from May 22, 2015) which is indicated by the dash-dot line in Fig. 4. By using the linear equation, the second correcting section 19 calculates the time correction amount in May 26, 2015 as 21 seconds (17 + 1 x 4). Then, the second correcting section 19 corrects the time of the internal clock 14 to be shifted by 21 seconds. As described above, the relational expression is calculated from time correction amounts for the internal clock 14, and therefore the time correction can be performed in accordance with the tendency of the deviation of the time of the internal clock 14. In the case where the degree of the time shift becomes larger (or smaller) according to the elapse of the time, particularly, the time can be accurately corrected by using a relational expression.

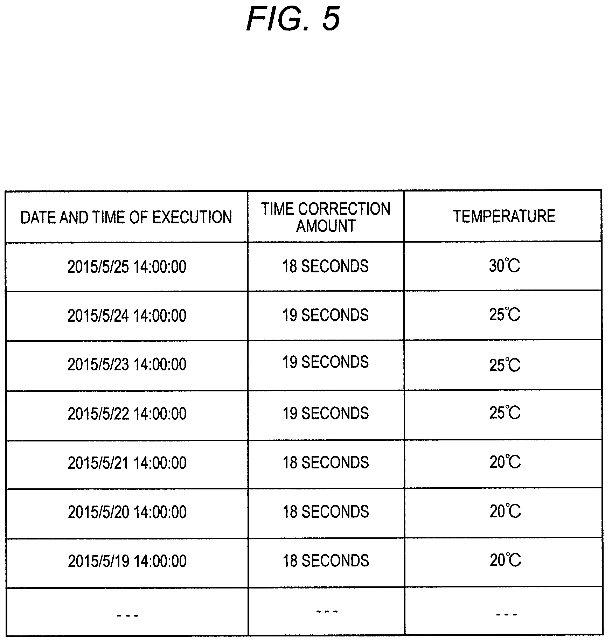

A fourth example will be described in which the relationship between the ambient temperature and the time deviation of the internal clock 14 is used. The crystal oscillator incorporated in the internal clock 14 is easily affected by the ambient temperature. Therefore, the first correcting section 18 stores the ambient temperature at the timing of the correction, and the time correction amount, in the storage section 15 while associating them with each other. The ambient temperature may be acquired from a thermometer (not illustrated in Fig. 1) disposed in the vital signs information measuring apparatus 1, or acquired from an external apparatus through the transmitter/receiver 12. Fig. 5 is a view illustrating the relationship between the ambient temperature and time correction amount which are stored in the storage section 15.

It is known that the frequency-temperature characteristic of a crystal oscillator usually exhibits a negative quadratic curve with the vertex at +25°C. The second correcting section 19 calculates the time correction amount in consideration of these data, the frequency-temperature characteristic of the crystal oscillator, and the current ambient temperature. The information of the current temperature may be acquired from the thermometer (not illustrated in Fig. 1) disposed in the vital signs information measuring apparatus 1, or acquired from an external apparatus through the transmitter/receiver 12. In the case where the temperature at 14 o'clock on May 26, 2015 is 25°C, for example, the second correcting section 19 calculates the current time correction amount as 19 seconds by using the history of the time correction amount shown in Fig. 5 (the time correction amount in May 22, 2015). Then, the second correcting section 19 performs correction on the internal clock 14 so as to shift the time by 19 seconds. In the case where the internal clock 14 uses a crystal oscillator, the ambient temperature functions as a significant cause of the time deviation. When the ambient temperature and the time correction amount are stored while being associated with each other as in the example, the time can be corrected in consideration of the ambient temperature.

Although the examples of the time correction by the second correcting section 19 have been described above, the time correction is not limited to the above-described four examples. The second correcting section 19 may perform time correction in which the history of the time correction amount stored by the first correcting section 18 is used.

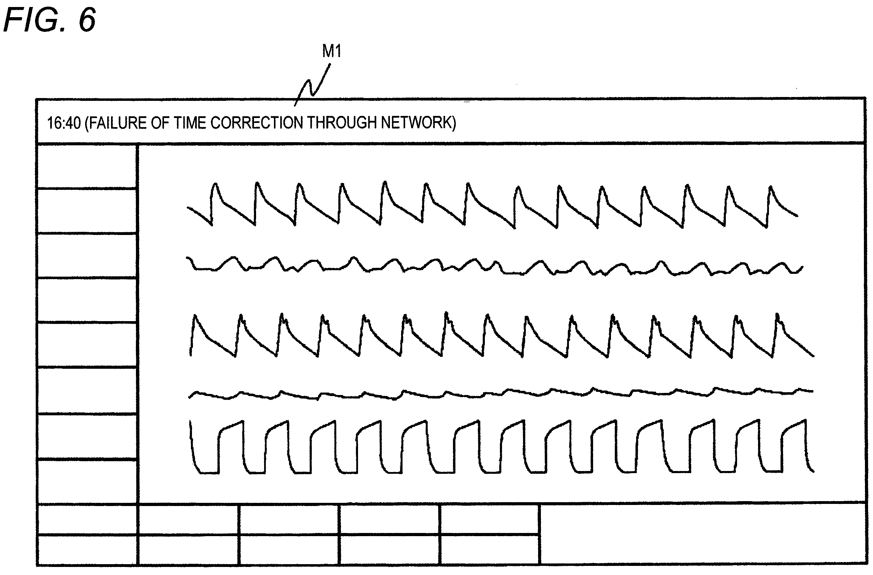

After the time correction, the second correcting section 19 may change the display screen on the displaying section 16. Fig. 6 is a view illustrating an example of the display screen which is displayed after the time correction is performed by the second correcting section 19. On the display screen, the message M1 "FAILURE OF TIME CORRECTION THROUGH NETWORK" is displayed adjacent to the display of the time. Namely, the displaying section 16 displays "Time correction has been performed by the second correcting section 19" (in other words, the displaying section 16 displays "The first correcting section 18 does not correctly perform time correction at a prescribed timing"). When the user (mainly the attending doctor, the nurse, the technologist, or the like) sees the display, the user can recognize that the desired time correction (for example, time correction through the network, i.e., time correction by the first correcting section 18) is not performed, and provisional time correction (time correction by the second correcting section 19) is performed.

The display shown in Fig. 6 is a mere example, and the display can be performed in any way as far as the user is informed that provisional time correction is performed. For example, the user may be informed by a display in which the color of the displayed time is changed to that different from the usual one, that in which the portion where the time is displayed is surrounded by a frame, or that in which the portion where the time is displayed is underscored. Even in the case of monochrome display or printing on a recording sheet, it can be informed that provisional time correction is performed, by a display in which the portion where the time is displayed is surrounded by a frame, that in which the portion where the time is displayed is underscored, or the like.

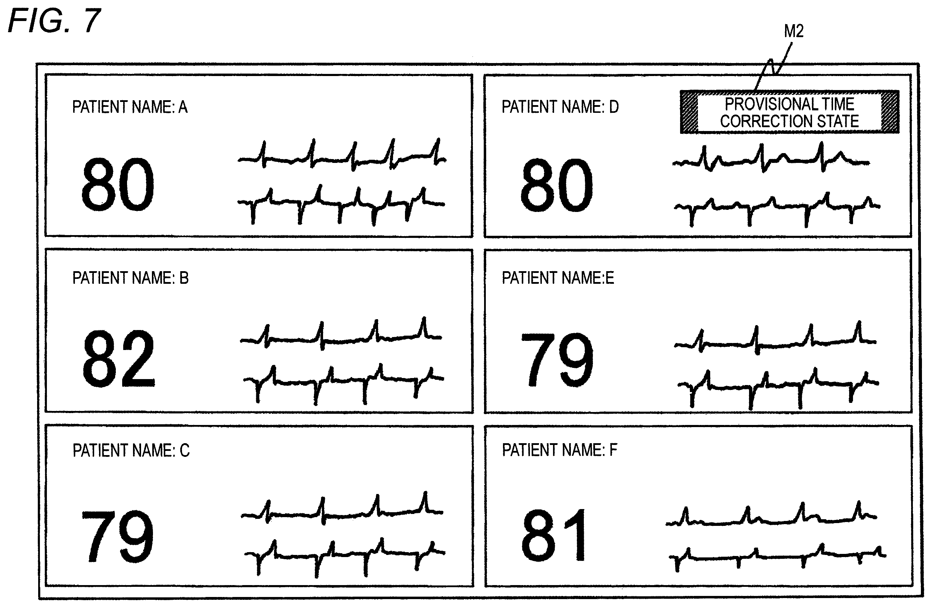

After the time correction, the second correcting section 19 may notify an external apparatus "The second correcting section 19 has corrected the time" (in other words, "The first correcting section 18 does not correctly perform time correction at a prescribed timing") through the transmitter/receiver 12. For example, the transmitter/receiver 12 notifies a central monitor that the second correcting section 19 has corrected the time. The central monitor displays a display window showing that the correction by the first correcting section 18 is not able to be performed, in a display window corresponding to the vital signs information measuring apparatus 1. Fig. 7 is a view showing an example of a display screen of the central monitor. In the example of Fig. 7, for example, the message M2 "PROVISIONAL TIME CORRECTION STATE" is displayed in a display window corresponding to the vital signs information measuring apparatus 1. The user (mainly the doctor, the nurse, or the like) can know that desired time correction (for example, time correction through the network) is not performed, and provisional time correction by the second correcting section 19 is performed. Therefore, the user can take countermeasures such as that the user requests to check problems of the network.

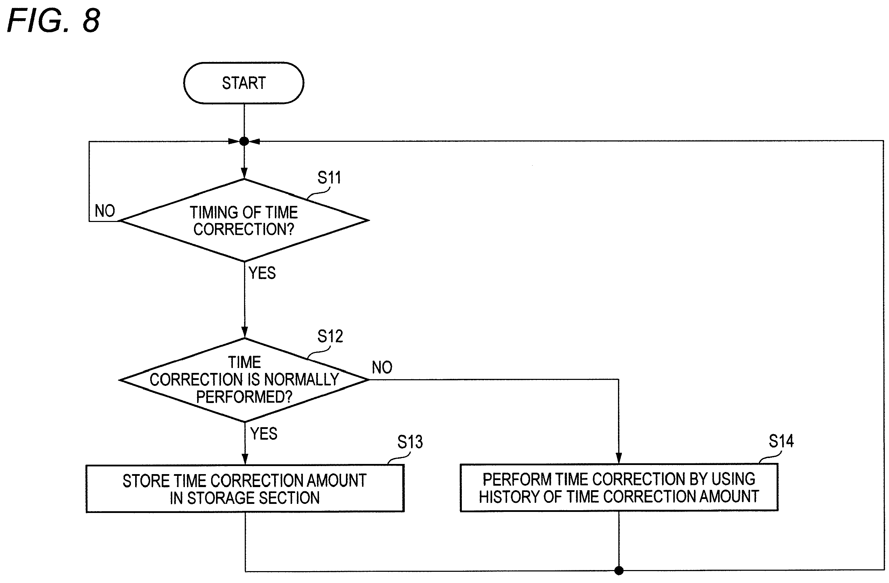

Then, the flow of the process of the controller 13 (the first correcting section 18 and the second correcting section 19) will be again described with reference to Fig. 8. Fig. 8 is a flowchart illustrating the process flow of the controller 13.

The first correcting section 18 waits for correction of the time of the internal clock 14 until the prescribed timing is reached (S11: No). When the prescribed timing is reached (S11: Yes), the first correcting section 18 tries to correct the time of the internal clock 14 by using the time information (S12). If the first correcting section 18 normally corrects the time of the internal clock 14 by using the time information (S12: Yes), the time correction amount is stored in the storage section 15 (S13).

By contrast, if the first correcting section 18 fails to normally correct the time of the internal clock 14 (S12: No), the second correcting section 19 corrects the time of the internal clock 14 by using the history of the time correction amount stored in the storage section 15 (S14). Although not illustrated in Fig. 8, after the correction process (S14) of the second correcting section 19, the change of the display screen (Fig. 6) and the notification to an external apparatus (Fig. 7) may be performed.

Then, effects of the vital signs information measuring apparatus 1 of the embodiment will be described. Firstly, the operation of a related-art vital signs information measuring apparatus will be described. Many related-art vital signs information measuring apparatuses have a function of correcting the time through a network. In the case where time correction is not performed for any reason, such an apparatus operates as it is. Namely, there is a possibility that the vital signs information measuring apparatus remains to be used in a state where time deviation occurs.

In the vital signs information measuring apparatus 1 of the embodiment, in the case where the first correcting section 18 fails to normally correct the time of the internal clock 14 at a prescribed timing, by contrast, the second correcting section 19 corrects the internal clock 14 by using the history of the time correction amount. Even in the case where desired time correction is not performed for any reason, therefore, an influence due to the time deviation of the internal clock 14 can be reduced. Moreover, the second correcting section 19 uses the history of the time correction amount which indicates the time deviation in the case where the first correcting section 18 has corrected the time by using the time information (information of the correct time). Therefore, the time can be accurately corrected.

In the case where the time correction has been performed by the second correcting section 19, it is possible to display the situation where provisional time correction is performed, on the displaying section 16 (Fig. 6). Therefore, the user can easily recognize that the time of the internal clock 14 is not corrected by a desired method. In the case of a practice in which the technologist inputs time information through the operating section 17, for example, the user can recognize forgetting to input or the like, and it is possible to quickly reestablish the practice method, or the like. In the case of a practice in which time information is acquired through the network, the user can easily know a network failure or the like relating to the vital signs information measuring apparatus 1.

Moreover, the transmitter/receiver 12 may inform an external apparatus that the time correction has been performed by the second correcting section 19. According to the configuration, the external apparatus (preferably, the central monitor) can know that the internal clock 14 of the vital signs information measuring apparatus 1 operates in the state where the time is provisionally corrected (Fig. 7). Even when the user is in a remote place such as a nurse station, therefore, the user can correctly know the situation of the internal clock 14.

Although the presently disclosed subject matter has been specifically described based on the embodiment, the presently disclosed subject matter is not limited to the above-described embodiment, and it is a matter of course that various changes can be made without departing from the spirit of the presently disclosed subject matter.

The processes of the first and second correcting sections 18, 19 in the controller 13 may be realized as computer programs which are executed in the vital signs information measuring apparatus 1.

The programs may be stored in a non-transitory computer readable medium of any one of various types, and then supplied to the computer. The non-transitory computer readable medium includes tangible storage media of various types. Examples of the non-transitory computer readable medium are a magnetic recording medium (for example, a flexible disk, a magnetic tape, and a hard disk drive), a magneto-optical recording medium (for example, a magneto-optical disk), a CD-ROM (Read Only Memory), a CD-R, a CD-R/W, a semiconductor memory (for example, a mask ROM, a PROM (Programmable ROM), an EPROM (Erasable PROM), a flash ROM, and a RAM (Random Access Memory)). Alternatively, the programs may be supplied to the computer by means of a transitory computer readable medium of any one of various types. Examples of the transitory computer readable medium include an electrical signal, an optical signal, and an electromagnetic wave. The transitory computer readable medium can supply the programs to the computer through a wired communication path such as a metal wire or an optical fiber, or a wireless communication path.

## Claims

1. A vital signs information measuring apparatus comprising:
an internal clock;
a first correcting section which is configured to execute first time correction on the internal clock by using time information, and which is configured to store a time correction amount in a storage section, the time correction amount indicating a degree of deviation of a time of the internal clock at a timing when the first time correction is performed by the first correcting section; and
a second correcting section which, in a case where it is detected that the first correcting section fails to normally correct the time of the internal clock at a prescribed timing, is configured to perform second time correction on the internal clock by using a history of the time correction amount stored in the storage section.

2. The vital signs information measuring apparatus according to claim 1, wherein
the second correcting section is configured to perform the second time correction on the internal clock by using an average of the history of the time correction amount.

3. The vital signs information measuring apparatus according to claim 1, wherein
the first correcting section is configured to store the time correction amount, and information relating to the timing when the first time correction is performed by the first correcting section, in the storage section, while associating the time correction amount and the information with each other, and
the second correcting section is configured to calculate a relational expression indicating a relationship between an elapsed time period and the deviation of the time of the internal clock, from data stored in the storage section, and is configured to perform the second time correction on the internal clock by using the relational expression.

4. The vital signs information measuring apparatus according to claim 1, wherein
the first correcting section is configured to store the time correction amount, and an ambient temperature at the timing when the first time correction is performed by the first correcting section, in the storage section, while associating the time correction amount and the ambient temperature with each other, and
the second correcting section is configured to perform the second time correction on the internal clock based on a current ambient temperature, and the time correction amount and the ambient temperature which are stored in the storage section.

5. The vital signs information measuring apparatus according to any one of claims 1 to 4, further comprising:
a displaying section which is configured to inform that the second time correction has been performed by the second correcting section.

6. The vital signs information measuring apparatus according to any one of claims 1 to 5, further comprising:
a transmitter which is configured to notify an external apparatus that the second time correction has been performed by the second correcting section.

7. A method of measuring vital signs information, the method comprising:
executing first time correction on an internal clock by using time information, and storing a time correction amount, the time correction amount indicating a degree of deviation of a time of the internal clock at a timing when the time correction is performed; and
in a case where it is detected that the first time correction is not normally performed at a prescribed timing, performing second time correction on the internal clock by using a history of the stored time correction amount.

8. A program which causes a computer to execute the method according to claim 7.

9. A non-transitory computer-readable recording medium in which a program causing a computer to execute the method according to claim 7 is recorded.
